(19) Europäisches Patentamt / European Patent Office / Office européen des brevets

(11) **EP 4 737 510 A1**

(12) **EUROPEAN PATENT APPLICATION**
published in accordance with Art. 153(4) EPC

(43) Date of publication:
06.05.2026 Bulletin 2026/19

(21) Application number: 24831769.5

(22) Date of filing: 18.06.2024

(51) International Patent Classification (IPC):
*C08J 11/24* (2006.01)   *C07C 69/82* (2006.01)

(52) Cooperative Patent Classification (CPC):
Y02W 30/62

(86) International application number:
**PCT/JP2024/022079**

(87) International publication number:
**WO 2025/004907 (02.01.2025 Gazette 2025/01)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: 30.06.2023 JP 2023108237

(71) Applicant: Rock Paint Co., Ltd.
Osaka-shi,
Osaka 555-0033 (JP)

(72) Inventors:
• EDA, Yasushi
Tokyo 136-0076 (JP)
• NAKAMURA, Natsumi
Tokyo 136-0076 (JP)
• MIZOGUCHI, Jun
Osaka-shi, Osaka 555-0033 (JP)
• MUKAI, Tomoya
Tokyo 136-0076 (JP)
• SHIRAI, Yu
Tokyo 136-0076 (JP)
• MITSUYASU, Hayato
Osaka-shi, Osaka 555-0033 (JP)
• YOSHIDA, Naoki
Osaka-shi, Osaka 555-0033 (JP)
• NAKAMURA, Toshio
Tokyo 136-0076 (JP)
• TSUNEKAWA, Yoshihide
Tokyo 136-0076 (JP)
• TAKANOHASHI, Yoshinori
Tokyo 136-0076 (JP)

(74) Representative: Marks & Clerk LLP
15 Fetter Lane
London EC4A 1BW (GB)

(54) **METHOD FOR RECYCLING POLYESTER-CONTAINING SUBSTANCE**

(57)     [Problem] To provide a method for recycling polyester-containing substances using a new means.

[Solution] A method for recycling polyester-containing substances, which includes a step of alcoholysis of polyester-containing substances in the presence of a catalyst and an alcohol in a solvent, and a step of separating the alcoholysis products obtained in the above-mentioned step. Specifically, the method is carried out using a device that has a tank for storing the polyester-containing substances and a tank and/or path for separating polyester alcoholysis products.

Fig. 1

EP 4 737 510 A1

**Description**

TECHNICAL FIELD

The present invention relates to a method for recycling a polyester-containing substance.

For completely decomposing a substance into its smallest units and purifying the decomposition products to remove impurities, not only it requires a significant amount of energy but also considerable effort.

Here, the term "recycling" in the present invention refers to making the above-mentioned polyester-containing substance into a state where it can be reused as a resource. That is, it is objected to reduce the energy consumption and effort required for recycling, and it is not necessarily intended to decompose and purify the substance into its smallest units through chemical decomposition. However, this does not exclude decomposition and purification that aims to reduce energy consumption and shortening processes, and for such purposes, the alcoholysis products of the above-mentioned polyester-containing substances obtained by the present invention can be used for chemical recycling.

The present invention is particularly useful for application to composites containing polyester components, and further, it is useful to use the components of the above-mentioned composites that remain unaffected by alcoholysis for material recycling.

BACKGROUND ART

Plastic is a material that is inexpensive and excellent in durability. In particular, polyethylene terephthalate (PET) has been widely used in various fields such as fibers, films, bottles, etc., due to its convenience, and it has been rapidly produced and consumed over the past few decades. On the other hand, despite its excellent durability, it is not easily decomposed, leading to the accumulation of plastic waste in landfills worldwide and exposing social issues of damage to the global environment such as marine pollution caused by microplastics, etc.

Given this background, it has been desired to develop recycling technologies for plastic waste, including PET. Compared to Europe and the United States, in Japan, a proportion of thermal recycling is higher, which involves utilizing thermal energy generated during incineration, but there remain concerns about global warming due to carbon dioxide emissions. Thus, extensive research has being conducted on chemical recycling of polyester products, that is, by converting and recovering polyester products into monomers through chemical means and reusing these monomers.

Patent document 1 discloses a method for producing aromatic divalent carboxylic acid dimethyl and dihydric alcohol, which are monomer components of aromatic polyesters such as PET, by continuous processing with use of supercritical alcohol. However, this method requires high temperatures of 300°C or higher.

Patent document 2 discloses a method for producing dimethyl terephthalate by subjecting to a depolymerization reaction of polyester with alkylene glycol, followed by a transesterification with methanol. However, this method requires to remove alkylene glycol at 150°C or higher by distillation.

Patent document 3 discloses a method for recycling a multilayer film containing a plastic layer primarily composed of polyester (PET), polypropylene (PP) and polyethylene (PE), and an aluminum layer. In this invention, the aluminum in the multilayer film waste is selectively dissolved to induce layer separation, and the mixture layer of PP and PE, and the PET layer are separated using difference in specific gravity. In addition, to enhance the purity of the PET separated by its difference in specific gravity, the PP and PE contained in the PET layer are extracted by using organic solvents, whereby the main components of the multilayer film are separated into the mixture of PET, PP and PE, and the aluminum component, respectively. However, since the aluminum is dissolved, energy is required for recycling.

Patent document 4 discloses a catalyst composition for polyester depolymerization comprising a base catalyst, a monohydric alcohol, and a carbonate diester or tetraalkoxysilane as a glycol scavenger, as well as a polyester depolymerization method using said catalyst composition. However, since ethylene glycol which is a constitutional component of PET is stably captured, the ethylene carbonate to be produced must be decomposed into ethylene glycol in order to reuse it again as a PET raw material.

Patent document 5 discloses a process for converting polyethylene terephthalate or poly(ethylene glycol-co-1,4-cyclohexanedimethanol terephthalate) into terephthalic acid esters by depolymerization. In a series of the process, a step of contacting the polyester with a solvent for swelling the polyester, an alcohol-based solvent, and a sub-stoichiometric amount of an alkoxide. In particular, in the examples, as the solvent for swelling the polyester, dichloromethane or dimethyl sulfoxide is used, methanol is used as the alcohol-based solvent, and sodium methoxide is used as the alkoxide in a sub-stoichiometric amount, and polyethylene terephthalate is converted into dimethyl terephthalate at 50 to 60°C. Here, the specification describes that the ratio of the solvent for swelling the above-mentioned polyester to the alcohol-based solvent is between about 0.5:1 and about 1:1 (w:w).

Patent document 6 discloses a method for depolymerizing poly(C2-C4 alkylene terephthalate). In particular, the specification discloses a method comprising a step of contacting the polymer with methanol and a catalyst selected from potassium carbonate, sodium carbonate, magnesium methoxide, 1,8-diazabicyclo-[5.4.0] undeca-7-ene, and triazabicyclodecene. Here, it is described that the above-mentioned depolymerization can be carried out at a temperature of about 100°C to about 180°C and a pressure of about 1 to 15 atmospheres.

Non-Patent document 1 discloses that, in the presence of an aluminum isopropoxide catalyst, dimethyl terephthalate and ethylene glycol can be obtained by methanolysis of polyethylene terephthalate (PET). Here, it is shown that the above-mentioned methanolysis is preferably carried out at 200°C, and a mixture of methanol and toluene is preferred. In particular, it is shown that by mixing and using the mixture such that the volume ratio of methanol to toluene is in the range of 8:2 to 5:5, the yield of dimethyl terephthalate and ethylene glycol can be 80% or higher.

PRIOR ART DOCUMENT

PATENT DOCUMENT

Patent document 1: JP 2001-039908A
Patent document 2: JP 2012-131729A
Patent document 3: JP 2006-205160A
Patent document 4: JP 2022-126617A
Patent document 5: JP 2020-533395A
Patent document 6: JP 2023-506948A

NON-PATENT DOCUMENT

Non-Patent document 1: Kurokawa et al., Methanolysis of polyethylene terephthalate in the presence of aluminium triisopropoxide catalyst to form dimethyl terephthalate and ethylene glycol, Polymer Degradation and Stability, 2003, 79, 529-533

SUMMARY OF THE INVENTION

PROBLEMS TO BE SOLVED BY THE INVENTION

The present invention was accomplished to overcome the problems of the prior art described above, and an object is to provide a method for recycling polyester-containing substances by new means.

MEANS TO SOLVE THE PROBLEM

The inventors of the present application conducted intensive research to solve the above-mentioned problem and found a method for recycling polyester-containing substances, which includes a step of alcoholysis of polyester-containing substances in the presence of a catalyst and an alcohol in a solvent, and a step of separating an alcoholysis product obtained in the above-mentioned step.

More specifically, the inventors of the present application have discovered a method for recycling polyester-containing substances, characterized by using a device that has a tank for storing the polyester-containing substances and a tank and/or a path for separating alcoholysis product of polyester, and thus have completed the present invention.

The polyester-containing substances (a) may be a polyester resin product, a composite containing polyester component, or may be a laminate formed by laminating a plurality of films.

The present invention can be applied to various polyesters as described below and is not particularly limited, but one example thereof, polyethylene terephthalate can be shown.

As for catalyst (b), as described below, various metal salts or nitrogen-containing organic base compounds can be used, and it is not particularly limited, but for example, at least one kind selected from the group consisting of alkali metal alkoxides and nitrogen-containing organic base compounds can be selected and implemented. More specifically, it may be shown sodium methoxide and/or 1,5,7-triazabicyclo[4.4.0]dec-5-ene as one example.

As described later, with respect to alcohol (c), it may be selected at least one kind from various types, and it is not particularly limited, but a monohydric alcohol having 1 to 8 carbon atoms is preferable, and methanol can be shown as only a specific example.

As described later, with respect to solvent (d), it is not particularly limited, but from the perspective of recycling, those which react with the alcoholysis products in the reaction system are excluded.

For example, at least one kind can be selected from the group of those that do not react with the alcoholysis products in the above-mentioned alcoholysis reaction system, and toluene can be shown as one specific example.

Also, according to the present invention, it is preferable to carry out a recycling method in such a way that, in addition to a step (A) of subjecting to alcoholysis of a polyester-containing substance (a) in the presence of a catalyst (b) and an alcohol (c) in a solvent (d), and a step (B) of separating an alcoholysis product obtained in the step (A), a step (C) of separating substances other than the alcoholysis product obtained in the step (A) is added.

The present invention could be provided a new means for recycling a polyester-containing substance (a).

Further, by carrying out the present invention, recycling a polyester-containing substance (a) can be sought without incinerating them, etc., thereby contributing to the reduction of carbon dioxide emissions.

BRIEF DESCRIPTION OF THE DRAWING

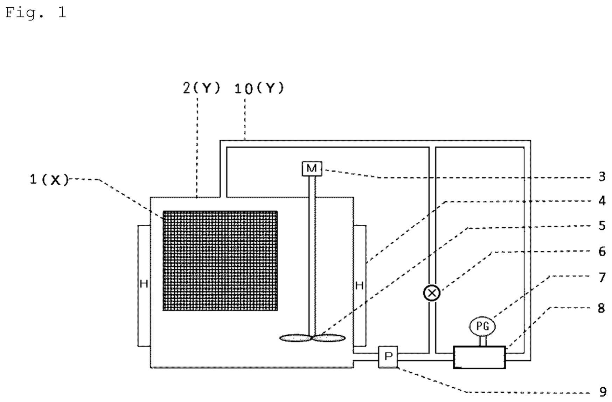

Fig. 1 is a structural diagram of a device for implementing the present invention.

EMBODIMENTS TO CARRY OUT THE INVENTION

The method for recycling a polyester-containing substance (a) by alcoholysis of the present invention comprises a catalyst (b) and an alcohol (c) and is carried out in a solvent (d). The formulation ratio of these components can be varied appropriately within the range in which the alcoholysis proceeds smoothly and implemented.

Hereinafter, each component used in the present invention will be explained in detail. Incidentally, the raw materials exemplified in the following explanation do not limit the constitution of the present invention, and may be modified within the scope of the present invention.

Polyester-containing substance (a)

The polyester-containing substance (a) of the present invention includes, as one embodiment, polyester resin products. As the representative polyester resin products, there may be mentioned polyethylene terephthalate (PET), amorphous polyethylene terephthalate (A-PET), glycol-modified polyethylene terephthalate (G-PET), polytrimethylene terephthalate (PTT), polybutylene terephthalate (PBT), polyethylene naphthalate (PEN), polybutylene naphthalate (PBN), polyarylate (PAR), polycaprolactone (PCL), polylactic acid (PLA), polyethylene adipate (PEA), and the like. These polyester resin products may be used alone or may be used in combination with two or more kinds.

In another embodiment, the polyester-containing substance (a) of the present invention may be a composite containing polyester components, and may include trays for food, fibers, adhesives, pressure-sensitive adhesives, inks, coatings, etc.

For example, in recent years, products in which polyamide resin has been combined with PET bottles to impart gas barrier properties have been adopted for wine beverages, etc., but even in such composites having physically additional substances, the present invention can be applied thereto. Since decomposition of the amide bonds cannot be carried out in the present invention, in the above-mentioned example, the polyethylene terephthalate, which is the raw material for PET bottles, is decomposed, while the polyamide resin remains without decomposition.

In addition, examples include those in which a vapor-deposited layer is provided on the surface of the polyester product or those with which coloring components are mixed for decorative purposes, and the present invention can be applied to all of these.

Further, the material may also include those in which a part of the components have been chemically modified.

Examples of the kinds of the resins used in polyester-based adhesives for laminate films may include polyester polyurethane, polyester polyether, polyester polyether polyurethane, etc., can be used.

Examples of the coating films formed by polyester-based coatings (polyester-based coating films) may include coating film formed by the oxidative polymerization of alkyd coating; polyester polyurethane coating film obtained by the reaction of polyester polyol and polyisocyanate; powder coating film formed by epoxy polyester-based powder coating or powder coating composed of a combination of a carboxyl group-containing polyester resin and a β-hydroxyalkylamide, etc.

Another embodiment of the polyester-containing substance (a) of the present invention also includes a laminate in which multiple films are laminated. There are no particular limitations on the films that can be used, but may be used polyester films such as PET; polyolefin films such as low-density polyethylene (LDPE), linear low-density polyethylene (LLDPE), high-density polyethylene (HDPE), biaxially oriented polypropylene (OPP), unoriented polypropylene (CPP); metal films such as aluminum foil (AL); nylon (NY) films; polyvinyl alcohol (PVA) films; cellulose films; acrylic films; polyphenylene sulfide (PPS) films; polyimide (PI) films; vinyl chloride-based films; fluorine resin-based films such as PTFE; and paper-based films. These may also be films on which vapor-deposited layers or ink layers are formed. In addition, colored films may also be used.

In the case where the polyester-containing substance (a) of the present invention is a laminate containing a polyester film, the polyester film is separated from other laminate films by alcoholysis, allowing for recycling. In addition, even in the case of a laminate that does not contain a polyester film, when the laminate is bonded with a polyester-based adhesive, the film bonded with the adhesive is separated by alcoholysis of the adhesive components, allowing for recycling. Those of which contain ester bonds in the films as exemplified above may be subject to alcoholysis in the present invention.

Thus, in the present invention, it is not necessary to decompose and purify the polyester-containing substance (a) down to the minimum monomer unit, and it is sufficient to decompose it to the extent that the components remaining without undergoing alcoholysis can be separated.

There is no particular limitation on the method of use of the adhesive used in the laminate in which the multiple films are laminated, and a general method of use as an adhesive for lamination can be applied. For example, there may be mentioned a non-solvent type lamination system in which the adhesive is used by heating to an appropriate viscosity, and a dry lamination system in which the adhesive is used by adding a dilution solvent or other formulating adhesive to adjust the coating viscosity to the appropriate level. An active energy ray-curable type adhesive may also be used. The coating amount is generally in the range of 1 to 10 $g/m^2$ in a dry state, but this may be appropriately changed depending on the type of the film and the performance requirements according to the application of the laminate. In addition, an aging period may also be set. For example, aging may be performed at 20°C or higher and 50°C or lower for 2 days or longer to 5 days or shorter.

The polyester-containing substance (a) can take various forms depending on the above-mentioned embodiments. For example, it can be used in a solid state such as a lump, fiber, film, pellet, etc. In addition, it can also be used in a liquid state.

Catalyst (b)

As the catalyst (b) of the present invention, a metal salt or a nitrogen-containing organic base compound can be used.

Examples of the metal salt that may include hydroxides, carbonates, fatty acid salts and alkoxides of alkali metals; hydroxides, carbonates, fatty acid salts, alkoxides and oxides of alkaline earth metals; and hydroxides, carbonates, fatty acid salts and alkoxides of transition metals. In particular, it is preferable to use alkali metal alkoxides and hydroxides of alkali metals. These catalysts may be used alone or in combination of two or more kinds.

Examples of the alkali metal alkoxides include lithium methoxide, lithium ethoxide, lithium tert-butoxide, sodium methoxide, sodium ethoxide, sodium tert-butoxide, potassium methoxide, potassium ethoxide, and potassium tert-butoxide. Among these, it is particularly preferable to use sodium methoxide.

Examples of the nitrogen-containing organic base compound include trimethylamine, triethylamine, tributylamine, pyrazole, imidazole, N-methylimidazole, benzimidazole, N-methylbenzimidazole, triazoles, benzotriazole, pyridine, quinoline, isoquinoline, triazines, 1,5,7-triazabicyclo[4.4.0]dec-5-ene (TBD), 1,8-azabicyclo[5.4.0]undec-7-ene (DBU) and 1,3-dimesitylimidazol-2-ylidene. Among these, it is particularly prefarable to use TBD.

Examples of the alkali metal hydroxides include lithium hydroxide, sodium hydroxide and potassium hydroxide. Among these, it is particularly preferable to use sodium hydroxide.

Incidentally, when an alkali metal hydroxide is used as the catalyst (b), the alcoholysis of the polyester component in the polyester-containing substance (a) tends to produce a large amount of by-products such as partial decomposition products. For example, when applied to a polyester resin product such as PET pellets, the reaction solution becomes a highly viscous liquid, which leads to require additional work for post-treatment, and thus it is not preferred from the viewpoint of recycling. On the other hand, for example, when applied to a polyester composite such as a laminate film composed of NY/polyester polyurethane adhesive/AL, the adhesive layer formed by the adhesive undergoes alcoholysis, and the interaction with the nylon film and aluminum foil that are not involved in alcoholysis is weakened. Therefore, this is advantageous for the separation of these constituent films, therefore it is preferable from the viewpoint of recycling.

The amount of the catalyst (b) added can be changed appropriately depending on the form of the polyester-containing substance (a), and is preferably 0.01 part by weight or more and 10 parts by weight or less based on 100 parts by weight of the polyester-containing substance (a), catalyst (b), alcohol (c) and solvent (d) in total. More preferably, it is 0.03 part by weight or more and 5 parts by weight or less.

The amount of catalyst (b) added should be adjusted within the range in which alcoholysis proceeds. However, when the amount added is large, not only will the cost increase, but it will also be more time-consuming to separate the catalyst residue. In addition, it is likely to have a negative effect on the components separated as substances other than the alcoholysis product, making material recycling difficult. On the other hand, when the amount added is small, there is a risk that alcoholysis will not proceed sufficiently due to the deactivation of the catalyst.

Alcohol (c)

The alcohol (c) of the present invention is not particularly limited, but is preferably a monohydric alcohol having 1 to 8 carbon atoms.

Examples of the monohydric alcohols include methanol, ethanol, 1-propanol, 2-propanol, 1-butanol, 2-butanol, tert-butanol, 2-ethylhexanol and benzyl alcohol. These alcohols may be used alone or in combination of two or more kinds. Among these, primary alcohols are preferred, and methanol is particularly preferred.

The amount of the alcohol (c) added can be changed appropriately depending on the form of the polyester-containing substance (a), and is preferably 0.9 part by weight or more and 50 parts by weight or less based on 100 parts by weight of the polyester-containing substance (a), catalyst (b), alcohol (c) and solvent (d) in total. More preferably, it is 2 parts by weight or more and 30 parts by weight or less.

The amount of the alcohol (c) added can be adjusted within the range in which alcoholysis proceeds. However, when the amount added is large, the cost increases. Also, if the amount added is small, alcoholysis does not proceed sufficiently.

Incidentally, in the present invention, it does not require complete decomposition of the polyester-containing substance (a) to the minimum unit and purification of the decomposition product, it is sufficient that alcoholysis proceeds to a degree sufficient for recycling. If the progress of alcoholysis is insufficient, alcoholysis can be resumed by adding more alcohol (c).

Solvent (d)

The solvent (d) of the present invention excludes those that react with the alcoholysis product in the alcoholysis reaction system.

For example, in the alcoholysis of polyethylene terephthalate, when dimethyl carbonate is selected as the solvent, ethylene glycol produced by the alcoholysis of polyethylene terephthalate reacts with dimethyl carbonate to convert to ethylene carbonate, thus, it is not suitable as a solvent from the viewpoint of recycling. In addition, when considering the reuse of the solvent, when the boiling point is too high, a lot of heat energy is required for the distillation operation, so the boiling point is preferably 200°C or less, more preferably 150°C or less, and even more preferably 120°C or less.

Examples of such solvents may include toluene, acetonitrile, N,N-dimethylformamide, dimethyl sulfoxide, n-hexane, cyclohexane, dichloromethane, tetrahydrofuran, methyl ethyl ketone and ethyl acetate. These solvents can be selected appropriately as long as they do not inhibit the alcoholysis, and may be used alone or in combination of two or more kinds. However, this does not include the use of the alcohol-based solvents as the solvent.

The ratio of the solvent (d) included in the present invention can be appropriately adjusted depending on the weight and shape of the polyester-containing substance (a). When the amount added is large, the reaction apparatus becomes large, as a result, the processing capacity of the polyester-containing substance (a) decreases. Furthermore, when the amount added is small, the contact efficiency between the catalyst (b), the alcohol (c) and the polyester-containing substance (a) becomes poor, leading to be difficult for alcoholysis to proceed sufficiently.

In the present invention, the mixing ratio of the alcohol (c) to the solvent (d) is preferably 1/1 to 1/99 by weight ratio. More preferably, it is 1/2 to 1/50 by weight ratio.

Other components (e)

In addition to the above (a) to (d), as other components, a drying agent or a substance not involved in alcoholysis may be contained. These do not limit the constitution of the present invention, and may be added as appropriate as long as it does not deviate the gist of the present invention.

Drying agent

In the drying agents, there are physical drying agents and chemical drying agents. These drying agents may be used alone or in combination of two or more kinds.

Physical drying agents include those that utilize the increased moisture and/or humidity transfer path due to the matrix structure inside the drying agent, and those that utilize physical interactions with the drying agent such as adsorption of moisture and/or humidity. Specific examples include silica gel, molecular sieves, zeolite, activated carbon, and the like.

Chemical drying agents include those that utilize to adsorb moisture and/or humidity by chemical reaction. Specific examples include metal oxides such as calcium oxide, barium oxide, magnesium oxide, and the like; metal salts such as sodium sulfate, calcium sulfate, magnesium sulfate, calcium chloride, magnesium chloride, sodium carbonate, potassium carbonate, and the like; diphosphorus pentoxide; silane compounds such as vinyltrimethoxysilane, dimethyldimethoxy-silane, methyltrimethoxysilane, methyltriethoxysilane, tetramethoxysilane, tetraethoxysilane, diphenyldimethoxysilane, phenyltrimethoxysilane, and the like; and orthoester compounds such as methyl orthoformate, ethyl orthoformate, methyl orthoacetate, ethyl orthoacetate, and the like. However, the chemical drying agents those of which react with the alcoholysis product are excluded from the viewpoint of recycling.

Substances not involved in alcoholysis

In the present invention, a substance not involved in alcoholysis may be included. Examples of films may include polyolefin films such as low-density polyethylene (LDPE), linear low-density polyethylene (LLDPE), high-density polyethylene (HDP), biaxially oriented polypropylene (OPP), cast polypropylene (CPP), and the like; metal films such as aluminum foil (AL); nylon (NY) film; cellulose film; polyphenylsulfide (PPS) film; polyimide (PI) film; vinyl chloride-based film; fluorine resin films such as PTFE. Paper films, polystyrene (PS) sheets, etc., may also be used. These films and sheets may have a vapor-deposited layer thereon. They may also be colored. They may be made by kneading a plurality of these components or by coextrusion.

Further, mineral components such as sodium chloride may be mixed therein.

It is preferable that the alcoholysis in the present invention proceed at a temperature of 20°C or higher and lower than 100°C, preferably 30°C or higher and lower than 90°C, and more preferably 40°C or higher and lower than 80°C. When the temperature is lower than 20°C, the catalytic activity is not sufficient. Although the alcoholysis proceeds at a temperature of 100°C or higher, this is not preferred from the viewpoint of energy costs.

The alcoholysis of the present invention can be carried out under normal pressure or reduced pressure, but may also be carried out under increased pressure.

The reaction time may be set the time required for the polyester-containing substance (a) to undergo alcoholysis, and may be set on, for example, 0.5 to 24 hours. The alcoholysis may not be allowed to proceed completely, and instead a separation step of the product may be carried out at an intermediate stage.

Step (A)

Step (A) of the present invention, in which a polyester-containing substance (a) is subjected to alcoholysis in a solvent (d) in the presence of a catalyst (b) and an alcohol (c), will be explained in detail below using a laminate of multiple different films containing a PET film as an example. Incidentally, the polyester-containing substance (a) can be washed and dried before use, if necessary.

The laminate as the polyester-containing substance (a), catalyst (b), alcohol (c) and solvent (d) are placed in a tank and heated while stirring. At this time, terephthalic acid derivatives and ethylene glycol derived from PET (hereinafter, these and catalyst residues are defined as "alcoholysis products") are obtained by alcoholysis. On the other hand, PET residues that remain unalcoholized and films other than PET (hereinafter, these are defined as "substances other than alcoholysis products") are also present in the tank.

Step (B)

The step (B), in which the alcoholysis product obtained in the step (A) is separated, will be explained.

When the alcoholysis product obtained in the step (A) is in a solid state, it can be separated by normal filtration or hot filtration. The filter used in the filtration operation is not particularly limited, and the size and material can be appropriately selected depending on the target.

When the alcoholysis product obtained in the step (A) is in a liquid state, it cannot be separated by filtration. Therefore, the separation can be achieved by, for example, a method of concentrating and recrystallizing or a method of extracting, but not limited to these methods.

In addition, water washing and liquid separation operations may be carried out as necessary.

The alcoholysis product obtained by these methods can be further decomposed and purified as necessary, and then subjected to chemical recycling.

Step (C)

To achieve the object of the present invention, it is preferable to include a step of separating substances other than the alcoholysis product in addition to the step (B).

In the example of the laminate described above, PET residues and films other than PET are mixed therein. In such cases, further separation can be carried out according to previously reported methods such as separation by specific gravity and dissolution in a specific solvent. Each of the separated constitutional films can be recycled.

Embodiment of the device

Next, referring to FIG. 1, one embodiment of the apparatus having a tank (X) and a tank and/or a path (Y) will be explained, but the various configuration of the apparatus can be adopted and implemented.

A polyester-containing substance (a) is placed in a tank 1 surrounded by a filter that does not allow any solids to pass therethrough, and a catalyst (b), an alcohol (c), and a solvent (d) are placed in a tank 2 and/or a path, and (a) to (d) are mixed so that they come into contact with each other. Here, the polyester-containing substance (a) in the tank 1 cannot pass through the filter surrounding the tank 1, but as alcoholysis progresses, the structure becomes such that only the alcoholysis product dissolved in the reaction solution can pass through the tank 1. A heater 4 may be provided in the tank 2 as necessary to adjust the reaction temperature.

The mixing methods may include stirring with a ceramic rotor, mechanical stirring with a stirring blade, and mixing by circulation. In the example of Fig. 1, an example of stirring with a stirring blade 5 at the tip of a rotating shaft connected to a motor 3 is shown. The liquid in the tank 2 and the liquid in the path connected to the tank 2 are circulated by a pump 9 provided in the middle of the path, and in this example, a valve 6 for adjusting the flow rate, a pressure gauge 7, and a filtration device 8 are provided in parallel in the path.

The materials of the apparatus used for recycling such as the tank 1, the tank 2 and/or the path can be selected appropriately as long as they do not hinder recycling.

The present invention can be carried out in the various embodiments as described above, as well as in the following embodiments.

The method for recycling a polyester-containing substance (a) according to this embodiment includes a step (A) of subjecting to alcoholysis of the polyester-containing substance (a) in a solvent (d) in the presence of a catalyst (b) and an alcohol (c), a step (B) of separating the alcoholysis product obtained in the step (A), and a step (C) of separating substances other than the alcoholysis product.

The alcohol (c) is a monohydric alcohol having 1 to 8 carbon atoms, and the amount of the alcohol (c) added can be 0.9 part by weight or more and 50 parts by weight or less based on the total of 100 parts by weight of the polyester-containing substance (a), the catalyst (b), the alcohol (c) and the solvent (d). More preferably, it can be 2 parts by weight or more and 30 parts by weight or less.

The solvent (d) contains toluene. The blending ratio of the alcohol (c) and the solvent (d) can be implemented by changing appropriately, but the amount of the solvent (d) is suitably 1 part by weight or more, preferably 2 parts by weight or more, and more preferably 2.1 parts by weight or more to per 1 part by weight of the alcohol (c). Furthermore, the amount of the solvent (d) is suitably 99 parts by weight or less, preferably 50 parts by weight or less, and more preferably 45.9 parts by weight or less to per 1 part by weight of the alcohol (c).

The alcoholysis is carried out at a temperature of 20°C or higher and lower than 100°C.

Also, it can be implemented that the alcohol (c) may be methanol, or the solvent (d) may be one that does not react with the alcoholysis product in the alcoholysis reaction system.

When implementing this embodiment, the same features as those of the previously described embodiments can be applied.

The polyester-containing substance (a) can be at least one kind selected from the group consisting of four kinds of polyethylene terephthalate, a polyester-based adhesive for a laminate film, a polyester-based coating film, and a laminate in which a plurality of films are bonded together using an adhesive.

The catalyst (b) is at least one kind selected from the group consisting of lithium methoxide, lithium ethoxide, lithium tert-butoxide, sodium methoxide, sodium ethoxide, sodium tert-butoxide, potassium methoxide, potassium ethoxide, potassium tert-butoxide, trimethylamine, triethylamine, tributylamine, pyrazole, imidazole, N-methylimidazole, benzimidazole, N-methylbenzimidazole, triazoles, benzotriazole, pyridine, quinoline, isoquinoline, triazines, 1,5,7-triazabicyclo[4.4.0]dec-5-ene (TBD), 1,8-azabicyclo[5.4.0]undec-7-ene (DBU) and 1,3-dimesitylimid-azol-2-ylidene.

In the case where the polyester-containing substance (a) is a laminate in which a plurality of films are bonded together via an adhesive, the catalyst (b) can be selected from at least one kind selected from the group consisting of lithium hydroxide, sodium hydroxide and potassium hydroxide in addition to the above-mentioned lithium methoxide, lithium ethoxide, lithium tert-butoxide, sodium methoxide, sodium ethoxide, sodium tert-butoxide, potassium methoxide, potassium ethoxide, potassium tert-butoxide, trimethylamine, triethylamine, tributylamine, pyrazole, imidazole, N-methylimidazole, benzimidazole, N-methylbenzimidazole, triazoles, benzotriazole, pyridine, quinoline, isoquinoline, triazines, 1,5,7-triaza-bicyclo[4.4.0]dec-5-ene (TBD), 1,8-azabicyclo[5.4.0]undec-7-ene (DBU) and 1,3-dimesitylimidazol-2-ylidene.

More specifically, it can be made that the polyester-containing substance (a) is a material containing polyethylene terephthalate, the catalyst (b) is sodium methoxide, the alcohol (c) is methanol, and the solvent (d) is toluene.

Also, it can be made that the polyester-containing substance (a) is a laminate in which a plurality of films are bonded together via an adhesive, at least one kind of plurality of the films is a PET film or a nylon film, the adhesive is a polyester-based adhesive, the catalyst (b) is sodium methoxide or sodium hydroxide, the alcohol (c) is methanol and the solvent (d) is toluene.

EXAMPLES

Hereinafter, the present invention will be described in more detail with reference to examples, but the present invention is not limited to the following examples.

In the examples shown below, the following reagents were used unless otherwise explained.

Polyester-containing substance (a)

·PET pellets (commercially available product)
·Eco A-PET film (commercially available product)
·G-PET flakes (commercially available product)
·Polyester polyurethane laminate adhesive (manufactured by Rock Paint Co., Ltd., AD ROCK RU-77/H-7), hereafter abbreviated as "AD1"
·Polyether polyurethane laminate adhesive (manufactured by Rock Paint Co., Ltd., AD ROCK RN-230/HN-230), hereafter abbreviated as "AD2"

Catalysts (b)

·Sodium methoxide (manufactured by FUJIFILM Wako Pure Chemical Corporation, Wako first grade)
·1,5,7-Triazabicyclo[4.4.0]dec-5-ene (TBD, manufactured by Tokyo Chemical Industry Co., Ltd.)
·Sodium hydroxide (manufactured by Kanto Chemical Co., Inc., Cica first grade)
·Potassium hydroxide (manufactured by Kanto Chemical Co., Inc.)
·Calcium hydroxide (manufactured by Kanto Chemical Co., Inc., special grade)

Alcohol (c)

·Methanol (manufactured by FUJIFILM Wako Pure Chemical Corporation, super-dehydrated grade)
·Ethanol (manufactured by FUJIFILM Wako Pure Chemical Corporation, super-dehydrated grade)
·Benzyl alcohol (manufactured by Tokyo Chemical Industry Co., Ltd.)
·Ethylene glycol (manufactured by FUJIFILM Wako Pure Chemical Corporation, dehydrated grade)

Solvent (d)

·Toluene (manufactured by Kanto Chemical Co., Inc., special grade)
·Ethyl acetate (manufactured by Kanto Chemical Co., Inc., special grade)
·Acetonitrile (manufactured by FUJIFILM Wako Pure Chemical Corporation, special grade)
·Tetrahydrofuran (manufactured by FUJIFILM Wako Pure Chemical Corporation, super-dehydrated grade (contains stabilizer))
·Dichloromethane (manufactured by Kanto Chemical Co., Inc., special grade)

Film

·PET film (manufactured by Toyobo Co., Ltd., Toyobo Ester Film E5102, 12 $\mu$m)
·OPP film (manufactured by Toyobo Co., Ltd., Pylen Film-OT P2241, 25 $\mu$m)
·CPP film (manufactured by Toyobo Co., Ltd., Pylen Film-CT P1146, 60 $\mu$m)
·Nylon (NY) film (manufactured by Unitika Ltd., Emblem ONBC-RT, 15 $\mu$m)
Aluminum foil (AL, manufactured by Toyo Aluminum K.K., 20 $\mu$m)
·LLDPE film (manufactured by RM Tohcello Co., Ltd., TUX-FC-S, 60 $\mu$m)

Incidentally, in the examples regarding laminated films, the above-mentioned films were used as an example, but the film thickness is not limited to these. However, it is preferable to appropriately adjust the amounts of the catalyst (b) and the alcohol (c) added depending on the film thickness.

Filter

·Mesh opening: 0.5 mm, made of stainless steel

With regard to the method for producing the laminated film, one example of a constitution comprising PET/AD1/OPP will be explained. After coating and drying the PET film with a bar coater so that the dry coating amount was 4 g/m$^2$, the OPP film was laminated and was nipped on a hot plate at 60°C. Thereafter, it was then aged at 40°C for three days.

The other constitutions were appropriately changed so that the dry coating amount was in the range of 1 to 10 g/m$^2$ depending on the type of film used and the use of the laminated film.

The progress of alcoholysis can be evaluated based on the alcoholysis rate A in the following formula (1):

Alcoholysis rate A [%] = (1 - weight of (a) after alcoholysis/weight of (a) before alcoholysis) x 100

In the examples shown below, the evaluation results are shown according to the following criteria.

Alcoholysis rate A is 90% or more: Excellent
Alcoholysis rate A is 60% or more and less than 90%: Good
Alcoholysis rate A is 30% or more and less than 60%: Average
Alcoholysis rate A is less than 30%: Poor

First, as a preliminary test, in Examples 1 to 18 and Comparative Examples 1 to 5, the alcoholysis rate A was verified using PETs, which are polyester resin products, among the polyester-containing substances (a).

[Example 1]

Sodium methoxide (1 part by weight) and methanol (2 parts by weight) were added to a tank in which PET pellets (6 parts by weight) were charged. Toluene was added so that the total of parts by weight became 100 parts by weight, and the mixture was stirred under the condition at 65°C for 2 hours.

After completion of the reaction, the reaction solution was filtered through a filter, and the filtration residue was washed with water. No unreacted PET pellets were obtained as the filtration residue. The alcoholysis rate A was 100%.

[Examples 2 to 18]

The same experiment under the condition of Example 1 was carried out, except that the conditions were changed to those shown in Tables 1 and 2.

[Comparative Example 1]

The same experiment under the condition of Example 1 was carried out, except that the sodium methoxide was changed to sodium hydroxide (1 part by weight). The alcoholysis rate A was 16.2%.

However, when sodium hydroxide was used as the catalyst (b), the reaction solution became a highly viscous liquid, and it was found that it was necessary to consider the adjustment of condition to efficiently proceed with the alcoholysis.

[Comparative Example 2]

The same experiment under the condition of Example 1 was carried out, except that the methanol was changed to ethylene glycol (4 parts by weight). The alcoholysis rate A was 0%.

[Comparative Example 3]

The same experiment under the condition of Example 1 was carried out, except that the methanol was changed to 80 parts by weight of the total amount. The alcoholysis rate A was 10.3%.

[Comparative Example 4]

The same experiment under the condition of Example 1 was carried out without adding sodium methoxide. The alcoholysis rate A was 0%.

[Comparative Example 5]

The same experiment under the condition of Example 1 was carried out without adding sodium methoxide and methanol. The alcoholysis rate A was 0%.

[Table 1]

| | | Example 1 | Example 2 | Example 3 | Example 4 | Example 5 | Example 6 | Example 7 | Example 8 | Example 9 | Example 10 | Example 11 |
|---|---|---|---|---|---|---|---|---|---|---|---|---|
| Polyester-containing substance (a) | PET pellet | 6.00 | 6.00 | 6.00 | 6.00 | 6.00 | 6.00 | 6.00 | | | 6.00 | 6.00 |
| | Eco A-PET | | | | | | | | 6.00 | | | |
| | G-PET flakes | | | | | | | | | 6.00 | | |
| Catalyst (b) | Sodium methoxide | 1.00 | 0.30 | 4.00 | 1.00 | 1.00 | 1.00 | 1.00 | 1.00 | 1.00 | | |
| | TBD | | | | | | | | | | 1.00 | 0.77 |
| | sodium hydroxide | | | | | | | | | | | |
| Alcohol (c) | Methanol | 2.00 | 2.00 | 2.00 | 0.93 | 10.00 | 40.00 | 46.50 | 2.00 | 2.00 | 2.00 | |
| | Ethanol | | | | | | | | | | | |
| | Benzyl alcohol | | | | | | | | | | | |
| | Ethylene glycol | | | | | | | | | | | 4.00 |
| Solvent (d) | Toluene | 91.00 | 91.70 | 88.00 | 92.07 | 83.00 | 53.00 | 46.50 | 91.00 | 91.00 | 91.00 | 89.23 |
| | Ethyl acetate | | | | | | | | | | | |
| | Acetonitrile | | | | | | | | | | | |
| | Tetrahydrofuran | | | | | | | | | | | |
| Substance not involved in alcoholysis | OPP film | | | | | | | | | | | |
| | Nylon film | | | | | | | | | | | |
| | Aluminum foil | | | | | | | | | | | |
| Total | | 100.00 | 100.00 | 100.00 | 100.00 | 100.00 | 100.00 | 100.00 | 100.00 | 100.00 | 100.00 | 100.00 |
| Alcohol (c)/Solvent (d) | | 1/45.5 | 1/45.9 | 1/44.0 | 1/99.0 | 1/8.3 | 1/1.3 | 1/1.0 | 1/45.5 | 1/45.5 | 1/45.5 | 1/22.3 |
| Reaction temperature (°C) | | 65 | 65 | 65 | 65 | 65 | 65 | 65 | 65 | 65 | 65 | 65 |
| Reaction time (hr) | | 2 | 2 | 2 | 2 | 2 | 2 | 2 | 2 | 2 | 2 | 2 |
| Judgement | | Excellent | Excellent | Excellent | Average | Excellent | Good | Good | Excellent | Good | Excellent | Good |
| Alcoholysis rate (%) | | 100.0 | 100.0 | 100.0 | 58.3 | 91.8 | 84.1 | 73.9 | 100.0 | 83.2 | 93.3 | 73.7 |

[Table 2]

| | | Example 12 | Example13 | Example 14 | Example 15 | Example 16 | Example 17 | Example 18 | Comparative Example 1 | Comparative Example 2 | Comparative Example 3 | Comparative Example 4 | Comparative Example 5 |
|---|---|---|---|---|---|---|---|---|---|---|---|---|---|
| Polyester-containing substance (a) | PET pellet | 6.00 | 6.00 | 6.00 | 6.00 | 6.00 | 6.00 | 6.00 | 6.00 | 6.00 | 6.00 | 6.00 | 6.00 |
| | Eco A-PET | | | | | | | | | | | | |
| | G-PET flakes | | | | | | | | | | | | |
| Catalyst (b) | Sodium methoxide | 1.00 | 1.00 | 1.00 | 1.00 | 1.00 | 1.00 | 1.00 | | 1.00 | 1.00 | | |
| | TBD | | | | | | | | | | | | |
| | Sodium hydroxide | | | | | | | | 1.00 | | | | |
| Alcohol (c) | Methanol | | | 2.00 | 2.00 | 2.00 | 2.00 | 2.00 | 2.00 | | 80.00 | 2.00 | |
| | Ethanol | | 3.00 | | | | | | | | | | |
| | Benzyl alcohol | 6.75 | | | | | | | | | | | |
| | Ethylene glycol | | | | | | | | | 4.00 | | | |
| Solvent (d) | Toluene | 86.25 | | | | 85.00 | 85.00 | 85.00 | 91.00 | 89.00 | 13.00 | 92.00 | 94.00 |
| | Ethyl acetate | | 90.00 | | | | | | | | | | |
| | Acetonitrile | | | 91.00 | | | | | | | | | |
| | Tetrahydrofuran | | | | 91.00 | | | | | | | | |
| Substance not involved in alcoholysis | OPP film | | | | | 6.00 | | | | | | | |
| | Nylon film | | | | | | 6.00 | | | | | | |
| | Aluminum foil | | | | | | | 6.00 | | | | | |
| Total | | 100.00 | 100.00 | 100.00 | 100.00 | 100.00 | 100.00 | 100.00 | 100.00 | 100.00 | 100.00 | 100.00 | 100.00 |
| Alcohol (c)/Solvent (d) | | 1/12.8 | 1/30.0 | 1/45.5 | 1/45.5 | 1/42.5 | 1/42.5 | 1/42.5 | 1/45.5 | 1/22.3 | 1/0.2 | 1/46.0 | - |
| Reaction temperature (°C) | | 65 | 65 | 65 | 60 | 65 | 65 | 65 | 65 | 65 | 65 | 65 | 65 |
| Reaction time (hr) | | 2 | 2 | 2 | 2 | 2 | 2 | 2 | 2 | 2 | 2 | 2 | 2 |
| Judgement | | Excellent | Excellent | Excellent | Excellent | Excellent | Excellent | Excellent | Poor | Poor | Poor | Poor | Poor |
| Alcoholysis rate (%) | | 93.0 | 98.5 | 99.7 | 94.0 | 99.7 | 97.9 | 100.0 | 16.2 | 0.0 | 10.3 | 0.0 | 0.0 |

EP 4 737 510 A1

13

As shown in Tables 1 and 2, it was confirmed that PET products, which are polyester resin products, underwent alcoholysis by appropriately combining the catalyst (b), alcohol (c) and solvent (d) of the present invention.

Incidentally, in Examples 16 to 18, it was confirmed that alcoholysis of PET pellets proceeded even when substances not involved in alcoholysis were present. After completion of the alcoholysis, OPP film, nylon film, and aluminum foil were recovered. In addition, these were not modified by the catalyst, alcohol or solvent of the present invention.

Next, in Examples 19 to 26, the alcoholysis rate A was verified using polyester composites among the polyester-containing substances (a).

[Example 19]

The same experiment under the condition of Example 1 was carried out, except that the PET pellets were changed to white PET film (manufactured by Toyobo Co., Ltd., Toyobo Ester Film E-5102 printed with white ink). The alcoholysis rate A was 95.9%.

[Example 20]

The same experiment under the condition of Example 1 was carried out, except that the PET pellets were changed to alumina transparent vapor-deposited PET film (manufactured by Toray Advanced Film Co., Ltd., Barrielox 1011SBR2). The alcoholysis rate A was 100%.

[Example 21]

The same experiment under the condition of Example 1 was carried out, except that the PET pellets were changed to polyester fibers (manufactured by Fujix Co., Ltd., King High Span Button Thread). The alcoholysis rate A was 90.0%.

[Example 22]

The same experiment under the condition of Example 1 was carried out, except that the PET pellets were changed to an aliphatic polyester resin (manufactured by Rock Paint Co., Ltd., AD ROCK RU-10). The alcoholysis rate A was 100%.

Incidentally, the polyester resin used here was dissolved in the reaction solution and could not be isolated by filtration, so that, after completion of the reaction, the reaction solution was isolated and characterized by GPC (gel permeation chromatography), and it was confirmed that no polyester resin which had not subjected to alcoholysis was present.

[Example 23]

The same experiment under the condition of Example 1 was carried out, except that the PET pellets were changed to a resin in which an epoxy resin (manufactured by Mitsubishi Chemical Group Corporation, jER1055K, 35 parts by weight) and a polyester resin (manufactured by Daicel-Allnex Ltd., CRYLCOAT 1713, 65 parts by weight) were crushed and mixed, and thermally cross-linked at 160°C for 20 minutes. These resins can be used as resins for powder coatings. For simplicity, pigments and other additives were not added, and the resin components alone were verified. The alcoholysis rate A was 45.8%.

[Example 24]

The same experiment under the condition of Example 1 was carried out, except that the PET pellets were changed to nylon-composite PET in which the PET pellets and nylon MXD6 (manufactured by Mitsubishi Gas Chemical Company, Inc., a crystalline polyamide obtained by polycondensation reaction of metaxylene diamine and adipic acid) were composited to bring a weight ratio of 95:5. The reaction solution was filtered through a filter, and the unreacted nylon-composite PET was removed from the resulting filter residue. The remaining material was analyzed using FT-IR (manufactured by Shimadzu Corporation, IRPrestige-21), and amide bonds originating from nylon MXD6 were detected. The alcoholysis rate A was 93.4%.

[Example 25]

Under the conditions of Example 20, a PET egg carton with a paper label was used instead of a laminated film.

After completion of the reaction, the reaction solution was filtered through a filter, the paper label was recovered, but no other filter residue other than the paper label was obtained. Here, the alcoholysis rate A was calculated as the PET egg carton thereof, excluding the paper label. The alcoholysis rate A was 100%.

[Example 26]

The same experiment under the condition of Example 20 was carried out, except by using a 65% polyester-35% cotton blend fiber instead of the laminated film. The alcoholysis rate A was 34.5%.

[Table 3]

| | | Example 27 | Example 28 | Example 29 | Example 30 | Example 31 | Example 32 | Example 33 | Example 34 | Example 35 | Comparative Example 6 |
|---|---|---|---|---|---|---|---|---|---|---|---|
| Polyester-containing substance (a) | PET/AD1/OPP | 6.00 | | | | | | | | | |
| | PET/AD1/CPP | | 6.00 | | | | | | | | |
| | PET/AD1/NY | | | 6.00 | | | | | | | |
| | PET/AD1/AL | | | | 6.00 | | | | | | |
| | NY/AD1/AL | | | | | 6.00 | | | | | |
| | PET/AD1/NY/AD1/AL | | | | | | 6.00 | | 6.00 | 6.00 | |
| | PET/AD2/NY | | | | | | | 6.00 | | | |
| | OPP/AD2/CPP | | | | | | | | | | 6.00 |
| Catalyst (b) | Sodium methoxide | 1.00 | 1.00 | 1.00 | 1.00 | 1.00 | 1.00 | 1.00 | 0.30 | 0.03 | 1.00 |
| Alcohol (c) | Methanol | 2.00 | 2.00 | 2.00 | 2.00 | 2.00 | 2.00 | 2.00 | 2.00 | 2.00 | 2.00 |
| Solvent (d) | Toluene | 91.00 | 91.00 | 91.00 | 91.00 | 91.00 | 91.00 | 91.00 | 91.70 | 91.97 | 91.00 |
| Total | | 100.00 | 100.00 | 100.00 | 100.00 | 100.00 | 100.00 | 100.00 | 100.00 | 100.00 | 100.00 |
| Alcohol (c)/Solvent (d) | | 1/45.5 | 1/45.5 | 1/45.5 | 1/45.5 | 1/45.5 | 1/45.5 | 1/45.5 | 1/45.5 | 1/45.5 | 1/45.5 |
| Reaction temperature (°C) | | 25 | 25 | 25 | 25 | 25 | 25 | 25 | 25 | 65 | 25 |
| Reaction time (hr) | | 4 | 4 | 4 | 4 | 4 | 4 | 4 | 4 | 2 | 4 |
| Judgement | | Excellent | Excellent | Excellent | Excellent | Excellent | Excellent | Excellent | Excellent | Excellent | Poor |
| Separation rate (%) | | 100.0 | 100.0 | 100.0 | 100.0 | 100.0 | 100.0 | 94.0 | 100.0 | 92.1 | 0.0 |

As shown in Table 3, by appropriately combining the catalyst (b), alcohol (c) and solvent (d) of the present invention, it was confirmed that even when a polyester composite was used as the material containing polyester (a), alcoholysis could be subjected.

Also, even in Example 23 in which nylon-composite PET was used as the polyester-containing substance (a), the nylon components that remained without undergoing alcoholysis could be separated, similar to Example 17 which used a mixture of nylon film.

Next, as the polyester-containing substance (a), alcoholysis when using laminated films having the constitutions described in Table 4 was investigated.

Here, the separation rate B was evaluated based on the following formula 2.

Separation rate B [%] = (1 - weight of unseparated laminated film after alcoholysis/weight of laminated film before alcoholysis) $\times$ 100          Formula 2

Separation rate B of 90% or higher: Excellent
Separation rate B of 60% or higher and less than 90%: Good
Separation rate B of 30% or higher and less than 60%: Average
Separation rate B of less than 30%: Poor

[Example 27]

A laminate film (6 parts by weight) composed of PET film/polyester-based adhesive (AD1)/OPP film was placed in a tank, and sodium methoxide (1 part by weight) and methanol (2 parts by weight) were added. Toluene was added to bring the total weight of 100 parts by weight, and the mixture was stirred under the condition at 25°C for 4 hours.

After completion of the reaction, the reaction solution was filtered through a filter, and the filter residue was washed with water, but the unseparated laminated film was not recovered. The separation rate B was 100%.

[Examples 28 to 34 and Comparative Example 6]

The same experiment under the condition of Example 20 was carried out, except by changing the conditions to those as shown in Table 4.

[Example 35]

A laminated film (6 parts by weight) composed of PET film/polyester-based adhesive (AD1)/nylon film/polyester-based adhesive (AD1)/aluminum foil was placed in a tank, and sodium methoxide (0.03 part by weight) and methanol (2 parts by weight) were added. Toluene was added to bring the total weight of 100 parts by weight, and the mixture was stirred under the condition at 65°C for 2 hours.

After completion of the reaction, the reaction solution was filtered through a filter, and the filter residue was washed with water to recover the unseparated laminate film. The separation rate B was 92.1%.

[Table 4]

| | | Example 27 | Example 28 | Example 29 | Example 30 | Example 31 | Example 32 | Example 33 | Example 34 | Example 35 | Comparative Example 6 |
|---|---|---|---|---|---|---|---|---|---|---|---|
| Polyester-containing substance (a) | PET/AD1/OPP | 6.00 | | | | | | | | | |
| | PET/AD1/CPP | | 6.00 | | | | | | | | |
| | PET/AD1/NY | | | 6.00 | | | | | | | |
| | PET/AD1/AL | | | | 6.00 | | | | | | |
| | NY/AD1/AL | | | | | 6.00 | | | | | |
| | PET/AD1/NY/AD1/AL | | | | | | 6.00 | | 6.00 | 6.00 | |
| | PET/AD2/NY | | | | | | | 6.00 | | | |
| | OPP/AD2/CPP | | | | | | | | | | 6.00 |
| Catalyst (b) | Sodium methoxide | 1.00 | 1.00 | 1.00 | 1.00 | 1.00 | 1.00 | 1.00 | 0.30 | 0.03 | 1.00 |
| Alcohol (c) | Methanol | 2.00 | 2.00 | 2.00 | 2.00 | 2.00 | 2.00 | 2.00 | 2.00 | 2.00 | 2.00 |
| Solvent (d) | Toluene | 91.00 | 91.00 | 91.00 | 91.00 | 91.00 | 91.00 | 91.00 | 91.70 | 91.97 | 91.00 |
| Total | | 100.00 | 100.00 | 100.00 | 100.00 | 100.00 | 100.00 | 100.00 | 100.00 | 100.00 | 100.00 |
| Alcohol (c)/Solvent (d) | | 1/45.5 | 1/45.5 | 1/45.5 | 1/45.5 | 1/45.5 | 1/45.5 | 1/45.5 | 1/45.5 | 1/45.5 | 1/45.5 |
| Reaction temperature (°C) | | 25 | 25 | 25 | 25 | 25 | 25 | 25 | 25 | 65 | 25 |
| Reaction time (hr) | | 4 | 4 | 4 | 4 | 4 | 4 | 4 | 4 | 2 | 4 |
| Judgement | | Excellent | Excellent | Excellent | Excellent | Excellent | Excellent | Excellent | Excellent | Excellent | Poor |
| Separation rate (%) | | 100.0 | 100.0 | 100.0 | 100.0 | 100.0 | 100.0 | 94.0 | 100.0 | 92.1 | 0.0 |

In Examples 27 to 30, the PET film and polyester-based adhesive (AD1) underwent alcoholysis, whereby the OPP film, CPP film, nylon film and aluminum foil were separated, respectively. None of these were modified by the catalyst, alcohol or solvent of the present invention.

In Example 31, the polyester-based adhesive (AD1) underwent alcoholysis, whereby the nylon film and aluminum foil were separated. None of these were modified by the catalyst, alcohol or solvent of the present invention.

In Example 32, the PET film and polyester-based adhesive (AD1) underwent alcoholysis, whereby the nylon film and aluminum foil were separated. None of these were modified by the catalyst, alcohol or solvent of the present invention.

In Example 33, the PET film underwent alcoholysis, whereby the nylon film was recovered. However, since the polyether-based adhesive (AD2) did not undergo alcoholysis, residual adhesive was observed in the recovered nylon film.

In Example 34, the catalyst amount was reduced to 0.3 parts by weight compared to Example 32, but the nylon film and aluminum foil were similarly separated.

In Example 35, the catalyst amount was reduced to 0.03 parts by weight compared to Example 32. Even with a small amount of catalyst, the nylon film and aluminum foil were similarly separated under conditions of 65°C.

In contrast, in Comparative Example 6, since a laminated film composed of components not involved in alcoholysis was used, each constituent film could not be separated.

Thus, it was found that by appropriately combining the catalyst (b), alcohol (c) and solvent (d), each constituent film of laminated films containing various polyester component can be efficiently separated.

[Examples 36 to 44 and Comparative Examples 7 to 9]

The same experiment under the condition of Example 27 was carried out, except by changing the conditions to those as shown in Table 5.

In Examples 36 to 44, since the catalyst (b) and the alcohol (c) were within the specified ranges, they were all separated into each constitutional film. Also, the separated films were not modified by the catalyst, alcohol or solvent of the present invention.

On the other hand, in Comparative Examples 7 to 9, where ethylene glycol was used as the alcohol (c), none of the constitutional films could be separated under the conditions of 25°C.

[Table 5]

| | | Example 36 | Example 37 | Example 38 | Example 39 | Example 40 | Example 41 | Example 42 | Example 43 | Example 44 | Comparative Example 7 | Comparative Example 8 | Comparative Example 9 |
|---|---|---|---|---|---|---|---|---|---|---|---|---|---|
| Polyester-containing substance (a) | PET/AD1/NY/AD1/LLDPE | 6.00 | | | 6.00 | | | 6.00 | | | 6.00 | | |
| | PET/AD1/NY/AD1/AL/AD1/CPP | | 6.00 | | | 6.00 | | | 6.00 | | | 6.00 | |
| | PET/AD1/AL/AD1/NY/AD1/CPP | | | 6.00 | | | 6.00 | | | 6.00 | | | 6.00 |
| Catalyst (b) | Sodium methoxide | 1.00 | 1.00 | 1.00 | 1.00 | 1.00 | 1.00 | 1.00 | 1.00 | 1.00 | 1.00 | 1.00 | 1.00 |
| | TBD | | | | | | | | | | | | |
| | Sodium hydroxide | | | | | | | | | | | | |
| | Potassium hydroxide | | | | | | | | | | | | |
| | Calcium hydroxide | | | | | | | | | | | | |
| Alcohol (c) | Methanol | 2.00 | 2.00 | 2.00 | 10.00 | 10.00 | 10.00 | 30.00 | 30.00 | 30.00 | | | |
| | EG | | | | | | | | | | 10.00 | 10.00 | 10.00 |
| Solvent (d) | Toluene | 91.00 | 91.00 | 91.00 | 83.00 | 83.00 | 83.00 | 63.00 | 63.00 | 63.00 | 83.00 | 83.00 | 83.00 |
| | Dichloromethane | | | | | | | | | | | | |
| | Total | 100.00 | 100.00 | 100.00 | 100.00 | 100.00 | 100.00 | 100.00 | 100.00 | 100.00 | 100.00 | 100.00 | 100.00 |
| | Alcohol (c)/Solvent (d) | 1/45.5 | 1/45.5 | 1/45.5 | 1/8.3 | 1/8.3 | 1/8.3 | 1/2.1 | 1/2.1 | 1/2.1 | 1/8.3 | 1/8.3 | 1/8.3 |
| | Reaction temperature (°C) | 25 | 25 | 25 | 25 | 25 | 25 | 25 | 25 | 25 | 25 | 25 | 25 |
| | Reaction time (hr) | 4 | 4 | 4 | 4 | 4 | 4 | 4 | 4 | 4 | 4 | 4 | 4 |
| | Judgement | Excellent | Excellent | Excellent | Excellent | Excellent | Excellent | Excellent | Excellent | Excellent | Poor | Poor | Poor |
| | Separation rate (%) | 100.0 | 100.0 | 100.0 | 100.0 | 100.0 | 100.0 | 100.0 | 100.0 | 100.0 | 0.0 | 0.0 | 0.0 |

[Examples 45 to 53]

The same experiment under the condition of Example 27 was carried out, except by changing the conditions to those as shown in Table 6.

In Examples 45 to 47, since the catalyst (b) and alcohol (c) were within the specified ranges, they were all separated into each constitutional film.

In Examples 48 to 50, the solvent (d) was changed from toluene to dichloromethane compared to Examples 45 to 47, and separation into each constitutional film could be performed in all cases.

In Examples 51 to 53, the catalyst (b) was changed from sodium methoxide to TBD compared to Examples 45 to 47, and separation into each constitutional film could be performed in all cases.

In addition, none of the separated films was not modified by the catalyst, alcohol or solvent of the present invention.

[Table 6]

| | | Example 45 | Example 46 | Example 47 | Example 48 | Example 49 | Example 50 | Example 51 | Example 52 | Example 53 |
|---|---|---|---|---|---|---|---|---|---|---|
| Polyester-containing substance (a) | PET/AD1/NY/AD1/PE | 6.00 | | | 6.00 | | | 6.00 | | |
| | PET/AD1/NY/AD1/AL/AD1/CPP | | 6.00 | | | 6.00 | | | 6.00 | |
| | PET/AD1/AL/AD1/NY/AD1/CPP | | | 6.00 | | | 6.00 | | | 6.00 |
| Catalyst (b) | Sodium methoxide | 0.30 | 0.30 | 0.30 | 0.30 | 0.30 | 0.30 | | | |
| | TBD | | | | | | | 0.77 | 0.77 | 0.77 |
| | Sodium hydroxide | | | | | | | | | |
| | Potassium hydroxide | | | | | | | | | |
| | Calcium hydroxide | | | | | | | | | |
| Alcohol (c) | Methanol | 10.00 | 10.00 | 10.00 | 10.00 | 10.00 | 10.00 | 10.00 | 10.00 | 10.00 |
| | EG | | | | | | | | | |
| Solvent (d) | Toluene | 83.70 | 83.70 | 83.70 | | | | 83.23 | 83.23 | 83.23 |
| | Dichloromethane | | | | 83.70 | 83.70 | 83.70 | | | |
| Total | | 100.00 | 100.00 | 100.00 | 100.00 | 100.00 | 100.00 | 100.00 | 100.00 | 100.00 |
| Alcohol (c)/Solvent (d) | | 1/8.4 | 1/8.4 | 1/8.4 | 1/8.4 | 1/8.4 | 1/8.4 | 1/8.3 | 1/8.3 | 1/8.3 |
| Reaction temperature (°C) | | 25 | 25 | 25 | 25 | 25 | 25 | 25 | 25 | 25 |
| Reaction time (hr) | | 4 | 4 | 4 | 4 | 4 | 4 | 4 | 4 | 4 |
| Judgement | | Excellent | Excellent | Excellent | Excellent | Excellent | Excellent | Excellent | Excellent | Excellent |
| Separation rate (%) | | 100.0 | 100.0 | 100.0 | 100.0 | 100.0 | 100.0 | 100.0 | 100.0 | 100.0 |

EP 4 737 510 A1

[Examples 54 to 63 and Comparative Examples 10 to 11]

The same experiment under the condition of Example 27 was carried out, except by changing the conditions to those as shown in Table 7.

In Examples 54 to 63, since the catalyst (b) and alcohol (c) were within the specified ranges, and separation into each constitutional film could be performed in all cases. Also, the separated films were not modified by the catalyst, alcohol or solvent of the present invention.

In Comparative Examples 10 to 11, the catalyst (b) was changed from sodium hydroxide to calcium hydroxide compared to Examples 56 to 57, and the separation of each constitutional film could not be performed.

[Table 7]

| | | Example 54 | Example 55 | Example 56 | Example 57 | Example 58 | Example 59 | Example 60 | Example 61 | Example 62 | Example 63 | Comparative Example 10 | Comparative Example 11 |
|---|---|---|---|---|---|---|---|---|---|---|---|---|---|
| Polyester-containing substance (a) | PET/AD1/NY/AD1/PE | 6.00 | | 6.00 | | 6.00 | | 6.00 | | 6.00 | | 6.00 | |
| | PET/AD1/NY/AD1/AL/AD1/CPP | | 6.00 | | 6.00 | | 6.00 | | 6.00 | | 6.00 | | 6.00 |
| | PET/AD1/AL/AD1/NY/AD1/CPP | | | | | | | | | | | | |
| Catalyst (b) | Sodium methoxide | | | | | | | | | | | | |
| | TBD | | | | | | | | | | | | |
| | Sodium hydroxide | 1.00 | 1.00 | 1.00 | 1.00 | 1.00 | 1.00 | 0.30 | 0.30 | | | | |
| | Potassium hydroxide | | | | | | | | | 1.00 | 1.00 | | |
| | Calcium hydroxide | | | | | | | | | | | 1.00 | 1.00 |
| Alcohol (c) | Methanol | 2.00 | 2.00 | 10.00 | 10.00 | 30.00 | 30.00 | 10.00 | 10.00 | 10.00 | 10.00 | 10.00 | 10.00 |
| | EG | | | | | | | | | | | | |
| Solvent (d) | Toluene | 91.00 | 91.00 | 83.00 | 83.00 | 63.00 | 63.00 | 83.70 | 83.70 | 83.00 | 83.00 | 83.00 | 83.00 |
| | Dichloromethane | | | | | | | | | | | | |
| Total | | 100.00 | 100.00 | 100.00 | 100.00 | 100.00 | 100.00 | 100.00 | 100.00 | 100.00 | 100.00 | 100.00 | 100.00 |
| Alcohol (c)/Solvent (d) | | 1/45.5 | 1/45.5 | 1/8.3 | 1/8.3 | 1/2.1 | 1/2.1 | 1/8.4 | 1/8.4 | 1/8.3 | 1/8.3 | 1/8.3 | 1/8.3 |
| Reaction temperature (°C) | | 25 | 25 | 25 | 25 | 25 | 25 | 25 | 25 | 25 | 25 | 25 | 25 |
| Reaction time (hr) | | 4 | 4 | 4 | 4 | 4 | 4 | 4 | 4 | 4 | 4 | 4 | 4 |
| Judgement | | Excellent | Excellent | Excellent | Excellent | Excellent | Excellent | Excellent | Excellent | Excellent | Excellent | Poor | Poor |
| Separation rate (%) | | 100.0 | 100.0 | 100.0 | 100.0 | 100.0 | 100.0 | 100.0 | 100.0 | 100.0 | 100.0 | 0.0 | 0.0 |

EP 4 737 510 A1

24

As shown in Tables 5 to 7, it was confirmed that by appropriately combining the catalyst (b), alcohol (c) and solvent (d) of the present invention, alcoholysis proceeded efficiently when laminate film was used as the polyester-containing substance (a), and each constitutional film could be separated.

EXPLANATION OF REFERENCE NUMERALS

1. Tank
2. Tank
3. Motor
4. Heater
5. Stirring blade
6. Valve
7. Pressure gauge
8. Filtration device
9. Pump

**Claims**

1. A method for recycling a polyester-containing substance (a), comprising:

   a step (A) of subjecting the polyester-containing substance (a) to alcoholysis in a solvent (d) in the presence of a catalyst (b) and an alcohol (c); and
   a step (B) of separating an alcoholysis product obtained in the step (A);
   wherein a weight ratio of the alcohol (c)/solvent (d) is 1/1 to 1/99, and the alcoholysis proceeds at a temperature of 20°C or higher and lower than 100°C.

2. The recycling method according to Claim 1,
   wherein the polyester-containing substance (a) is at least one kind selected from the group consisting of four kinds of a polyethylene terephthalate, a polyester-based adhesive for a laminate film, a polyester-based coating film, and a laminate in which a plurality of films are bonded together with an adhesive.

3. The recycling method according to Claim 1,
   wherein the catalyst (b) is at least one kind selected from the group consisting of an alkali metal alkoxide and a nitrogen-containing organic basic compound.

4. The recycling method according to Claim 1,
   wherein the catalyst (b) is sodium methoxide and/or 1,5,7-triazabicyclo[4.4.0]dec-5-ene.

5. The recycling method according to Claim 1,
   wherein the alcohol (c) is a monohydric alcohol having 1 to 8 carbon atoms, and the amount is 2 to 30 parts by weight to the total amount of 100 parts by weight of the polyester-containing substance (a), the catalyst (b), the alcohol (c) and the solvent (d).

6. The recycling method according to Claim 1,
   wherein the alcohol (c) is methanol.

7. The recycling method according to Claim 1,
   wherein the solvent (d) does not react with the alcoholysis product in alcoholysis reaction system.

8. The recycling method according to Claim 1,
   wherein the solvent (d) contains toluene.

9. The recycling method according to Claim 1,
   wherein the polyester-containing substance (a) is a laminate in which a plurality of films are bonded together with a polyester-based adhesive for a laminate film, and the catalyst (b) is an alkali metal hydroxide.

10. The recycling method according to Claim 9,
    wherein the catalyst (b) is sodium hydroxide.

11. The recycling method according to any one of Claims 1 to 10, further comprising
    a step (C) of separating substance other than the alcoholysis product.

12. The recycling method according to Claim 11,
    wherein a device that is provided with a tank (X) for storing the polyester-containing substance (a) and a tank and/or
    path (Y) for separating the polyester alcoholysis product is used.

Fig. 1

## INTERNATIONAL SEARCH REPORT

| International application No. |
| --- |
| **PCT/JP2024/022079** |

**A. CLASSIFICATION OF SUBJECT MATTER**

*C08J 11/24*(2006.01)i; *C07C 69/82*(2006.01)i
FI: C08J11/24 ZAB; C07C69/82

According to International Patent Classification (IPC) or to both national classification and IPC

**B. FIELDS SEARCHED**

Minimum documentation searched (classification system followed by classification symbols)

C08J11/24; C07C69/82

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X | CN 114904542 A (QINGDAO INSTITUTE OF BIOENERGY AND BIOPROCESS TECHNOLOGY, CHINESE ACADEMY OF SCIENCES) 16 August 2022 (2022-08-16) claims, paragraphs [0001]-[0029], [0171]-[0176] | 1-4, 6-8, 11-12 |
| Y | | 9-12 |
| X | EP 4183823 A1 (KOREA RESEARCH INSTITUTE OF CHEMICAL TECHNOLOGY) 24 May 2023 (2023-05-24) claims, paragraphs [0069]-[0082], [0093] | 1-2, 5-8, 11-12 |
| Y | | 9-12 |
| X | JP 2020-533395 A (9449710 CANADA INC.) 19 November 2020 (2020-11-19) claims, paragraphs [0057]-[0186] | 1-2, 4-7, 11-12 |
| Y | | 9-12 |
| Y | WO 2022/220543 A1 (KOREA RESEARCH INSTITUTE OF CHEMICAL TECHNOLOGY) 20 October 2022 (2022-10-20) paragraphs [0153]-[0154] | 9-12 |

☐ Further documents are listed in the continuation of Box C. ☑ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **29 July 2024** | **20 August 2024** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (July 2022)

## INTERNATIONAL SEARCH REPORT
### Information on patent family members

International application No.

**PCT/JP2024/022079**

| Patent document cited in search report | | | Publication date (day/month/year) | Patent family member(s) | | | Publication date (day/month/year) |
|---|---|---|---|---|---|---|---|
| CN | 114904542 | A | 16 August 2022 | (Family: none) | | | |
| EP | 4183823 | A1 | 24 May 2023 | WO | 2022/060153 | A1 | |
| JP | 2020-533395 | A | 19 November 2020 | US<br>claims, paragraphs [0036]-[0161]<br>WO | 2019/0084916<br><br>2019/051597 | A1<br><br>A1 | |
| WO | 2022/220543 | A1 | 20 October 2022 | EP<br>paragraph [0118] | 4299556 | A1 | |

Form PCT/ISA/210 (patent family annex) (July 2022)

**REFERENCES CITED IN THE DESCRIPTION**

**Patent documents cited in the description**

- JP 2001039908 A
- JP 2012131729 A
- JP 2006205160 A

- JP 2022126617 A
- JP 2020533395 A
- JP 2023506948 A

**Non-patent literature cited in the description**

- **KUROKAWA et al.** Methanolysis of polyethylene terephthalate in the presence of aluminium triisopropoxide catalyst to form dimethyl terephthalate and ethylene glycol. *Polymer Degradation and Stability*, 2003, vol. 79, 529-533